(19) Europäisches Patentamt
European Patent Office
Office européen des brevets

(11) Veröffentlichungsnummer: **0 010 674**
**B1**

# (12) EUROPÄISCHE PATENTSCHRIFT

(45) Veröffentlichungstag der Patentschrift: **27.01.82**

(51) Int. Cl.³: **C 07 D 249/08**, C 07 D 233/60, A 01 N 43/50, A 01 N 43/64

(21) Anmeldenummer: **79103966.2**

(22) Anmeldetag: **15.10.79**

(54) **Neue Ethen-azol-Derivate, Verfahren zu ihrer Herstellung, fungizide Mittel, Verfahren zur Herstellung dieser sowie Verwendung der Azol-Derivate zur Bekämpfung von Pilzen und ein Verfahren zur Bekämpfung von Pilzen.**

(30) Priorität: **28.10.78 DE 2846980**

(43) Veröffentlichungstag der Anmeldung: **14.05.80 Patentblatt 80/10**

(45) Bekanntmachung des Hinweises auf die Patenterteilung: **27.01.82 Patentblatt 82/4**

(84) Benannte Vertragsstaaten: **AT BE CH DE FR GB IT NL SE**

(56) Entgegenhaltungen:
**DE-A1-2 634 511**
**DE-A1-2 652 313**
**DE-A1-2 737 489**
**US-A-3 972 892**
**US-A-4 066 361**

(73) Patentinhaber: **BAYER AG, Zentralbereich Patente, Marken und Lizenzen, D-5090 Leverkusen 1, Bayerwerk (DE)**

(72) Erfinder: **Jäger, Gerhard, Dr., Gellertstrasse 18, D-5090 Leverkusen 1 (DE)**
Erfinder: **Kraatz, Udo, Dr., Körner Strasse 6, D-5090 Leverkusen 1 (DE)**
Erfinder: **Büchel, Karl Heinz, Prof., Dr., Bergerheide 62, D-5600 Wuppertal 1 (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen 1 (DE)**

**EP 0 010 674 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue Ethen-azol-Derivate, Verfahren zu ihrer Herstellung, fingizide Mittel, Verfahren zur Herstellung dieser sowie Verwendung der Azol-Derivate zur Bekämpfung von Pilzen und ein Verfahren zur Bekämpfung von Pilzen

Die vorliegende Erfindung betrifft neue 1-Ethen-azol-Derivate, ein Verfahren zu ihrer Herstellung und ihre Verwendung als Fungizide.

Es ist bereits bekanntgeworden, daß 1-Ethyl-imidazol- und -triazol-Derivate, wie insbesondere im Phenylteil substituierte 1-(Imidazol-1-yl)- bzw. (1,2,4-Triazol-1-yl)-1-phenoxy-4,4-dimethyl-pentan-3-one gute fungizide Eigenschaften aufweisen (vgl. DE-OS 2 306 495 und DE-OS 2 335 002). Deren Wirkung ist jedoch, insbesondere bei niedrigen Aufwandmengen und -konzentrationen, nicht immer ganz befriedigend.

Es wurden neue 1-Ethylen-azol-Derivate der allgemeinen Formel

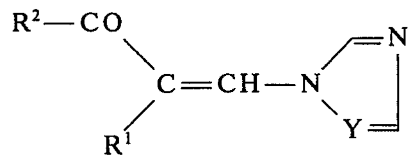

(I)

in welcher

- $R^1$ für Phenyl oder Phenoxy steht, wobei die genannten Reste substituiert sein können durch Halogen, Alkyl mit 1 bis 4 Kohlenstoffatomen, Halogenalkyl mit 1 bis 2 Kohlenstoff- und 1 bis 5 Fluor- oder Chloratomen, sowie durch gegebenenfalls durch Halogen substituiertes Phenyl,
- $R^2$ für Alkyl mit 1 bis 4 Kohlenstoffatomen, Cycloalkyl mit 5 bis 7 Kohlenstoffatomen, sowie für Phenyl steht, welches substituiert sein kann durch Halogen, Alkyl mit 1 bis 4 Kohlenstoffatomen, Halogenalkyl mit 1 bis 2 Kohlenstoff- und 1 bis 5 Fluor- oder Chloratomen, sowie durch gegebenenfalls durch Halogen substituiertes Phenyl, und
- Y für ein Stickstoffatom oder die CH-Gruppe steht,

und deren physiologisch verträglichen Säureadditionssalze und Metallkomplexe gefunden.

Die Verbindungen der Formel (I) können in zwei geometrischen Isomerenformen vorliegen, je nach Anordnung der Gruppen, welche an die Kohlenstoffatome gebunden sind, die durch die Doppelbindung verbunden sind. Sowohl die Isomeren als auch die Gemisches beider werden erfindungsgemäß beansprucht.

Man erhält die 1-Ethen-azol-Derivate der Formel (I), wenn man 1-Halogen-ethen-Derivate der Formel

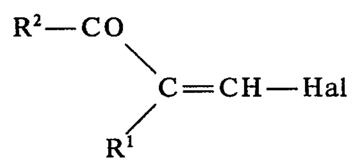

(II)

in welcher

- $R^1$ und $R^2$ die oben angegebene Bedeutung haben und
- Hal für Halogen steht,

mit Alkalisalzen von Azolen der Formel

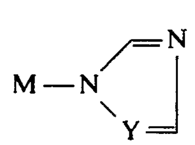

(III)

in welcher

- Y die oben angegebene Bedeutung hat und
- M für ein Alkalimetall steht,

in Gegenwart eines Verdünnungsmittels umsetzt.

Weiterhin können die erfindungsgemäß erhältlichen 1-Ethen-azol-Derivate der Formel (I) durch Umsetzen mit Säuren in die Salze überführt werden, bzw. können durch Reaktion mit Metallsalzen die entsprechenden Metallsalz-Komplexe erhalten werden. In manchen Fällen erweist es sich als

vorteilhaft, die Verbindungen der Formel (I) über ihre Salze in reiner Form zu erhalten.

Die neuen 1-Ethen-azol-Derivate weisen starke fungizide Eigenschaften auf. Überraschenderweise zeigen die erfindungsgemäßen Verbindungen eine erhebliche höhere fungizide Wirksamkeit als die aus dem Stand der Technik bekannten 1-Ethyl-imidazol- und -triazol-Derivate, wie insbesondere im Phenylteil substituierte 1-(Imidazol-1-yl)- bzw. -(1,2,4-Triazol-1-yl)-phenoxy-4,4-dimethylpentan-3-one, welche chemisch und wirkungsmäßig naheliegende Verbindungen sind. Die erfindungsgemäßen Stoffe stellen somit eine Bereicherung der Technik dar.

Die erfindungsgemäßen 1-Ethen-azol-Derivate sind durch die Formel (I) allgemein definiert.

Bevorzugt sind diejenigen Verbindungen der Formel (I), in denen $R^1$ für jeweils gegebenenfalls einfach oder mehrfach, gleich oder verschieden durch Fluor, Chlor, Brom, Methyl, Ethyl oder Phenyl substituiertes Phenyl oder Phenoxy steht, $R^2$ für Methyl, Isopropyl, tert.-Butyl, Cyclohexyl, sowie für gegebenenfalls einfach oder mehrfach, gleich oder verschieden durch Fluor, Chlor, Brom, Methyl, Ethyl oder Phenyl substituiertes Phenyl steht, und Y für ein Stickstoffatom oder die CH-Gruppe steht.

Im einzelnen seien außer den bei den Herstellungsbeispielen genannten Verbindungen die folgenden Verbindungen der allgemeinen Formel (I) genannt:

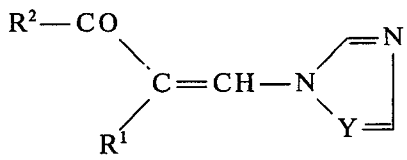

| $R^1$ | $R^2$ | Y |
|---|---|---|
| 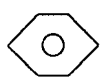 | 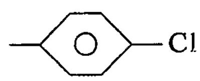 | N |
| 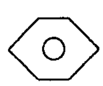 | 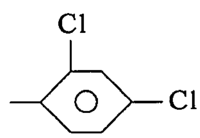 | N oder CH |
| 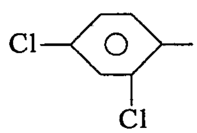 | 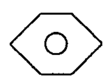 | N oder CH |
| 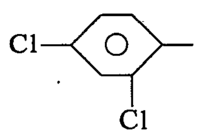 | 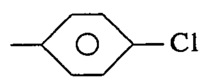 | N oder CH |
| 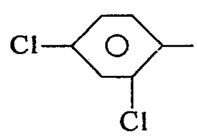 | 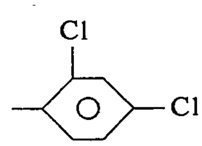 | N oder CH |
| 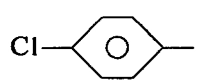 | 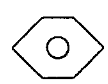 | N oder CH |
| 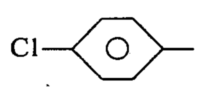 | 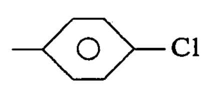 | N oder CH |
| 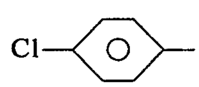 | 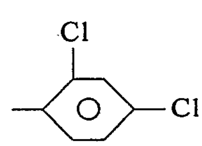 | N oder CH |

Chlorwasserstoffsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommem vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich von physiologisch verträglichen Säuren ableiten. Hierzu gehören vorzugsweise die Halogenwasserstoffsäuren, wie z. B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z. B. durch Lösen des Metallsalzes in Alkohol, z. B. Äthanol, und Hinzufügen zur Verbindung der Formel (I). Man kann Metallsalzkomplexe in bekannter Weise, z. B. durch Abfiltrieren, Isolieren und gegebenenfalls durch Umkristallisieren reinigen.

Die erfindungsgemäßen Wirkstoffe weisen eine starke fungizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z. B. gegen den Erreger des Gurkenmehltaus (Erysiphe cichoracearum) oder des Getreidemehltaus (Erysiphe graminis), sowie zur Bekämpfung von Venturia-Arten, wie z. B. gegen den Erreger des Apfelschorfs (Fusicladium dendriticum), und zur Bekämpfung von Puccinia-Arten, wie z. B. gegen den Erreger des Getreiderosts (Puccinia recondita). Gute Wirkungen werden auch gegen Pyricularia oryzae und Pellicularia sasakii am Reis erzielt.

In entsprechenden Aufwandkonzentrationen zeigen die erfindungsgemäßen Stoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulaten aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und

Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteteten Anwendungsformen, wie gebrauchsfertige Lösungen, Emulsionen, Suspensionen, Pulver, Pasten und Granulaten angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,0001 bis 0,1 Gew.-%, vorzugsweise von 0,0001 bis 0,02%, am Wirkungsort erforderlich.

## Beispiel A

### Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gew.-Teilen Dimethylformamid und 0,06 Gewichtsteilen Emulgator (Alkyl-aryl-polyglykol-ether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei. Nach 6 Tagen Verweilzeit der Pflanzen bei einer Temperatur von 21 bis 22° C und einer Luftfeuchtigkeit von 80 bis 90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Wirkstoffe, Wirkstoffkonzentrationen in der Spritzbrühe und Befallsgrade werden ermittelt. Bei diesem Test zeigen z. B. folgende Verbindungen eine sehr gute Wirkung, die derjenigen der aus dem Stand der Technik bekannten Verbindung überlegen ist:

Verbindungen gemäß Herstellungsbeispielen 3, 4, 1, 10.

## Beispiel B

### Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator Alkylaryl-polyglykolether auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 Stunden bei etwa 20° C und einer 100%igen Luftfeuchtigkeit in ein Gewächshaus.

Nach 10 Tagen Verweilzeit der Pflanzen bei einer Temperatur von 20° C und einer Luftfeuchtigkeit von 80 bis 90% wertet man den Besatz der Pflanzen mit Rostpusteln aus. Der Befallsgrad wird in

(V-1) 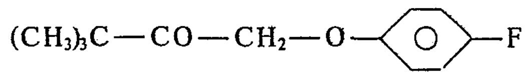

418,3 g (3,11 Mol) 2,2-Dimethyl-4-chlor-butan-3-on werden in eine zum Sieden erhitzte Suspension von 315 g (2,8 Mol) 4-Fluorphenol und 386,4 g (2,8 Mol) Kaliumcarbonat in 1500 ml Aceton getropft. Man läßt 4 Stunden unter Rückfluß rühren. Nach Abkühlung auf Raumtemperatur wird das ausgeschiedene Salz abfiltriert und das Filtrat im Vakuum eingeengt. Das zurückbleibende Öl wird im Vakuum destilliert. Man erhält 101,5 g (86,2% der Theorie) 2,2-Dimethyl-4-(4-fluorphenoxy)-butan-3-on vom Siedepunkt 83—84° C/0,05 mm ($n_D^{20}$ = 1,4919).

Beispiel 2

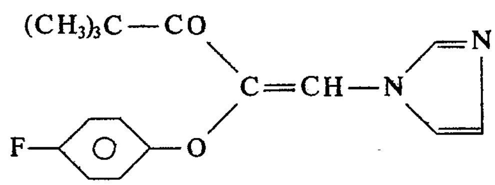

20 g (0,057 Mol) 1-(Imidazol-1-yl)-2-(4-fluorphenoxy)-4,4-dimethyl-penten-3-on-nitrat (erhalten gemäß Beispiel 1) werden in Wasser suspendiert. Die Suspension wird mit 10%iger Natriumcarbonatlösung alkalisch gestellt. Durch Extraktion der wäßrig-alkalischen Phase mit Chloroform erhält man nach den üblichen Aufarbeitungsmethoden 16,4 g (100% der Theorie) 1-(Imidazol-1-yl)-2-(4-fluorphenoxy)-4,4-dimethyl-penten-3-on vom Brechungsindex $n_D^{20}$ = 1,5590.

In entsprechender Weise werden die nachfolgenden Verbindungen der allgemeinen Formel

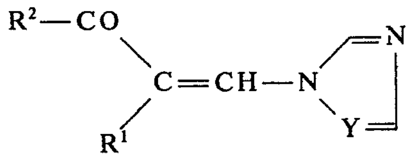 (I)

erhalten:

| Bsp. Nr. | $R^1$ | $R^2$ | Y | Schmelzpunkt °C |
|---|---|---|---|---|
| 3 | 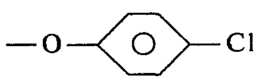 | $C(CH_3)_3$ | CH | 93–4 |
| 4 | 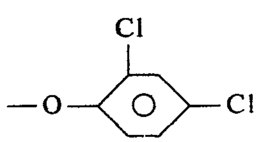 | $C(CH_3)_3$ | CH | 98–9 |
| 5 | 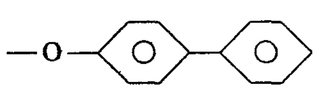 | $C(CH_3)_3$ | CH | 117–18 |
| 6 | 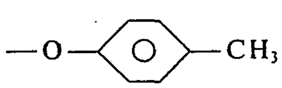 | $C(CH_3)_3$ | CH | 104–05 |
| 7 | 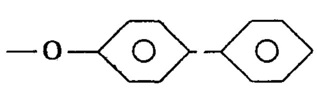 | $C(CH_3)_3$ | CH | 164 (Zers.) (x $HNO_3$) |
| 8 | 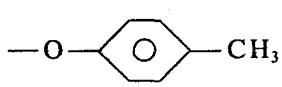 | $C(CH_3)_3$ | CH | 145 (Zers.) (x $HNO_3$) |
| 9 | 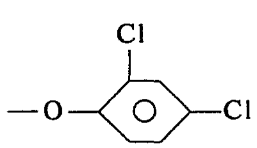 | $C(CH_3)_3$ | CH | 132 (Zers.) (x $HNO_3$) |

Fortsetzung

| Bsp. Nr. | $R^1$ | $R^2$ | Y | Schmelzpunkt °C |
|---|---|---|---|---|
| 10 | 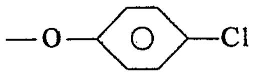 | $C(CH_3)_3$ | CH | 144 (Zers.) (x $HNO_3$) |
| 11 | 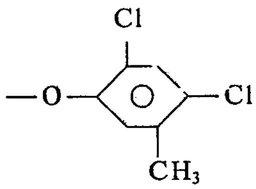 | $C(CH_3)_3$ | CH | 128–29 |
| 12 | 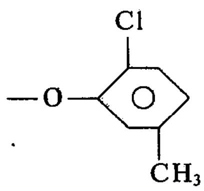 | $C(CH_3)_3$ | CH | 153 (Zers.) (x $HNO_3$) |
| 13 | 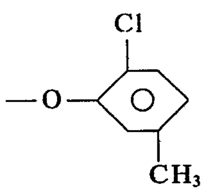 | $C(CH_3)_3$ | CH | 250–252 (Zers.) (x 0,5 Naphthalin-1,5-disulfonsäure) |
| 14 | 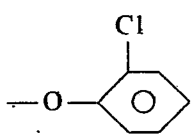 | $C(CH_3)_3$ | CH | 115–16 |
| 15 | 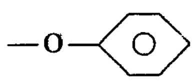 | $C(CH_3)_3$ | CH | 110–11 |
| 16 | 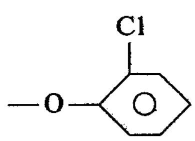 | $C(CH_3)_3$ | CH | 145 (Zers.) (x $HNO_3$) |
| 17 | 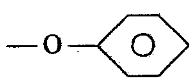 | $C(CH_3)_3$ | CH | 202–05 (x HCl) |
| 18 | 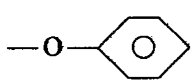 | $C(CH_3)_3$ | CH | 140 (Zers.) (x $HNO_3$) |
| 19 | 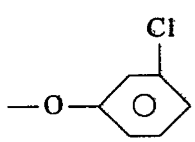 | $C(CH_3)_3$ | CH | 123 (Zers.) (x $HNO_3$) |
| 20 | 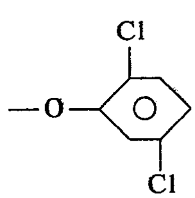 | $C(CH_3)_3$ | CH | 152 (Zers.) (x $HNO_3$) |

Fortsetzung

| Bsp. Nr. | $R^1$ | $R^2$ | Y | Schmelzpunkt °C |
|---|---|---|---|---|
| 45 | 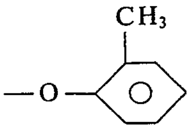 | $C(CH_3)_3$ | N | 87–88 |
| 46 | 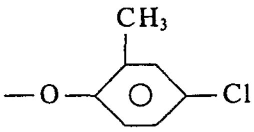 | $C(CH_3)_3$ | CH | 111–12 |
| 47 | 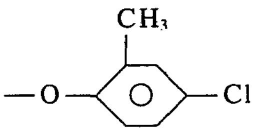 | $C(CH_3)_3$ | N | 98–100 |

Nach bekanntem Verfahren und entsprechend dem Herstellungsbeispiel für die Vorstufe (II-1) (siehe dort) werden die Ausgangsprodukte der allgemeinen Formel

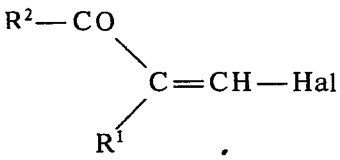

(II)

erhalten:

| Bsp. Nr. | $R^1$ | $R^2$ | Hal | Siedepunkt (°C)/mm Hg-Säule<br>Schmelzpunkt °C |
|---|---|---|---|---|
| II-2 | 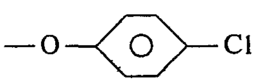 | $C(CH_3)_3$ | Cl | 115–17/0,1 |
| II-3 | 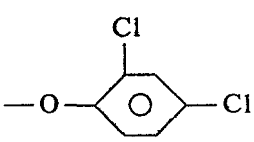 | $C(CH_3)_3$ | Cl | 135–37/0,07 |
| II-4 | 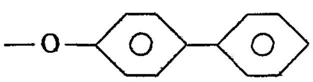 | $C(CH_3)_3$ | Cl | Öl |
| II-5 | 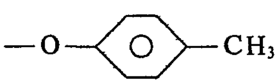 | $C(CH_3)_3$ | Cl | 114–16/0,4 |
| II-6 | 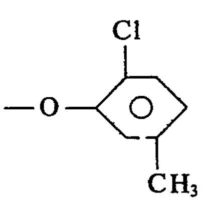 | $C(CH_3)_3$ | Cl | 121–23/0,4 |
| II-7 | 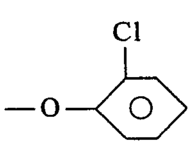 | $C(CH_3)_3$ | Cl | 107–10/0,15 |

Fortsetzung

| Bsp. Nr. | $R^1$ | $R^2$ | Hal | Siedepunkt (°C)/mm Hg-Säule<br>Schmelzpunkt °C |
|---|---|---|---|---|
| II-8 | 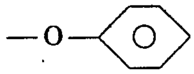 | $C(CH_3)_3$ | Cl | 98–100/0,25 |
| II-9 | 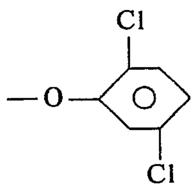 | $C(CH_3)_3$ | Cl | 132/0,3 |
| II-10 | 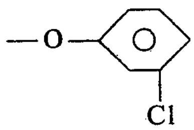 | $C(CH_3)_3$ | Cl | 126–30/0,2 |
| II-11 | 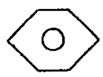 | 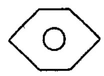 | Cl | Fp. 60–61 (reines Isomer) |
| II-12 | 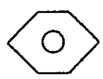 | 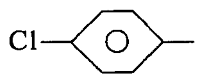 | Cl | Fp. 79–80 (reines Isomer) |
| II-13 | 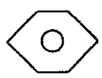 | 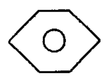 | Cl | 142–45/0,2 |

in welcher

Y die oben angegebene Bedeutung hat und
M für ein Alkalimetall steht,

in Gegenwart eines Verdünnungsmittels umsetzt, und gegebenenfalls noch die erhaltenen 1-Ethen-azol-Derivate durch Umsetzen mit Säuren in die Salze bzw. durch Reaktion mit Metallsalzen in die entsprechenden Metallsalz-Komplexe überführt.

3. Fungizide Mittel, gekennzeichnet durch einen Gehalt an mindestens einem Ethen-azol-Derivat gemäß Ansprüchen 1 und 2.

4. Verwendung von Ethen-azol-Derivaten gemäß Ansprüchen 1 und 2 zur Bekämpfung von Pilzen.

5. Verfahren zur Herstellung von fungiziden Mitteln, dadurch gekennzeichnet, daß man Ethen-azol-Derivate gemäß Ansprüchen 1 und 2 mit Streckmitteln und/oder oberflächenaktiven Mitteln vermischt.

**Claims**

1. Ethylene-azole derivatives corresponding to the following general formula

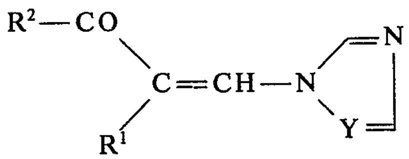

(I)

in which

$R^1$ represents phenyl or phenoxy radicals which may be substituted by halogen, $C_1-C_4$-alkyl, $C_1-C_2$-halogen alkyl containing from 1 to 5 fluorine or chlorine atoms, and by phenyl optionally substituted by halogen,
$R^2$ represents alkyl containing from 1 to 4 carbon atoms, $C_5-C_7$-cycloalkyl and phenyl which may be substituted by halogen, $C_1-C_4$-alkyl, $C_1-C_2$-halogen alkyl containing from 1 to 5 fluorine or chlorine atoms, and by phenyl optionally substituted by halogen, and
Y represents a nitrogen atom or the CH-group,

and their physiologically compatible acid addition salts and metal salt complexes.

2. A process for the production of ethylene-azole derivatives corresponding to the following general formula

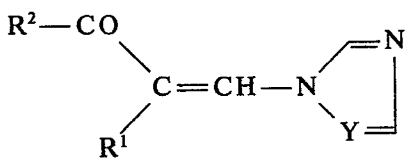

(I)

in which

$R^1$ represents phenyl and phenoxy radicals which may be substituted by halogen, $C_1-C_4$-alkyl, $C_1-C_2$-halogen alkyl containing from 1 to 5 fluorine or chlorine atoms, and by phenyl optionally substituted by halogen,
$R^2$ represents $C_1-C_4$-alkyl, $C_5-C_7$-cycloalkyl and phenyl which may be substituted by halogen, $C_1-C_4$-alkyl, $C_1-C_2$-halogen alkyl containing from 1 to 5 fluorine or chlorine atoms, and by phenyl optionally substituted by halogen, and
Y represents a nitrogen atom or the CH-group,

and their physiologically compatible acid addition salts and metal salt complexes, characterised in that 1-halogen ethylene derivatives corresponding to the following formula

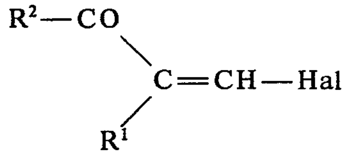

(II)

in which

$R^1$ and $R^2$ are as defined above and
Hal represents halogen,

are reacted with alkali salts of azoles corresponding to the following formula

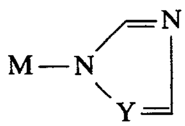

in which

Y is as defined above and
M represents an alkali metal,

in the presence of a diluent and the 1-ethylene-azole derivatives obtained are optionally converted into the salts by reaction with acids or into the corresponding metal salt complexes by reaction with metal salts.

3. Fungicidal compositions containing at least one ethylene-azole derivative of the type claimed in Claims 1 and 2.

4. The use of the ethylene-azole derivatives claimed in Claims 1 and 2 for controlling fungi.

5. A process for the production of fungicidal compositions, characterised in that ethylene-azole derivatives of the type claimed in Claims 1 and 2 are mixed with extenders and/or surfactants.

**Revendications**

1. Dérivés d'éthène-azoles de formule générale:

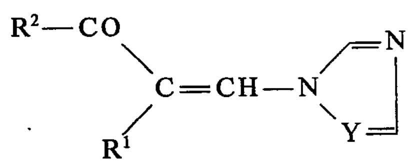
(I)

dans laquelle

$R^1$ représente un groupe phényle ou un groupe phénoxy, ces groupes pouvant être substitués par un atome d'halogène, par un groupe alkyle contenant 1 à 4 atomes de carbone, par un groupe halogénalkyle contenant 1 ou 2 atomes de carbone et 1 à 5 atomes de fluor ou de chlore, ainsi que par un groupe phényle éventuellement substitué par un atome d'halogène,
$R^2$ représente un groupe alkyle contenant 1 à 4 atomes de carbone, un groupe cycloalkyle contenant 5 à 7 atomes de carbone, de même qu'un groupe phényle pouvant être substitué par un atome d'halogène, par un groupe alkyle contenant 1 à 4 atomes de carbone, par un groupe halogénalkyle contenant 1 ou 2 atomes de carbone et 1 à 5 atomes de fluor ou de chlore, ainsi que par un groupe phényle éventuellement substitué par un atome d'halogène, et
Y représente un atome d'azote ou le groupe CH,

de même que les sels d'addition d'acide et les complexes de sels métalliques physiologiquement compatibles de ces dérivés.

2. Procédé de préparation de dérivés d'éthène-azoles de formule générale:

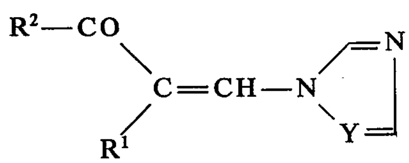
(I)

dans laquelle

$R^1$ représente un groupe phényle ou un groupe phénoxy, ces groupes pouvant être substitués par un atome d'halogène, par un groupe alkyle contenant 1 à 4 atomes de carbone, par un groupe halogénalkyle contenant 1 ou 2 atomes de carbone et 1 à 5 atomes de fluor ou de chlore, ainsi que

par un groupe phényle éventuellement substitué par un atome d'halogène,

$R^2$ représente un groupe alkyle contenant 1 à 4 atomes de carbone, un groupe cycloalkyle contenant 5 à 7 atomes de carbone, de même qu'un groupe phényle pouvant être substitué par un atome d'halogène, par un groupe alkyle contenant 1 à 4 atomes de carbone, par un groupe halogénalkyle contenant 1 ou 2 atomes de carbone et 1 à 5 atomes de fluor ou de chlore, ainsi que par un groupe phényle éventuellement substitué par un atome d'halogène, et

Y représente un atome d'azote ou le groupe CH,

ainsi que des sels d'addition d'acide et des complexes de sels métalliques physiologiquement compatibles de ces dérivés, caractérisé en ce qu'on fait réagir des dérivés de 1-halogéno-éthène de formule:

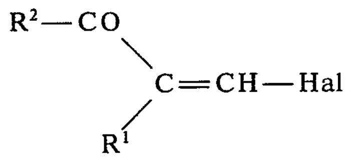

(II)

dans laquelle

$R^1$ et $R^2$ ont les significations indiquées ci-dessus, et
Hal représente un atome d'halogène,

avec des sels alcalins d'azoles de formule:

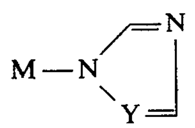

(III)

dans laquelle

Y a la signification indiquée ci-dessus, et
M représente un métal alcalin,

en présence d'un diluant,
et l'on transforme encore éventuellement les dérivés de 1-éthène-azoles obtenus en sels par réaction avec des acides, ou en complexes de sels métalliques correspondants par réaction avec des sels métalliques.

3. Agents fongicides, caractérisés en ce qu'ils contiennent au moins un dérivé d'éthène-azole suivant les revendications 1 et 2.

4. Utilisation de dérivés d'éthène-azoles suivant les revendications 1 et 2 pour combattre les champignons.

5. Procédé de préparation d'agents fongicides, caractérisé en ce qu'on mélange des dérivés d'éthène-azoles suivant les revendications 1 et 2 avec des diluants et/ou des agents tensio-actifs.